# EUROPEAN PATENT APPLICATION

(11) **EP 4 295 753 A1**
(43) Date of publication of application: **27.12.2023**
(21) Application number: 22180891.8
(22) Date of filing: 24.06.2022
(51) Int. Cl.: A61B 5/00, A61B 5/024, A61B 5/1455

(54) **OPTICAL SENSOR SYSTEM AND METHOD FOR MEASURING PHYSIOLOGICAL PARAMETERS**

(71) Applicant: Stichting IMEC Nederland, 6708 WH Wageningen (NL)
(72) Inventor: Tourolle, Duncan, 6862 AV Oosterbeek (NL); van Kraaij, Alex, 6532 CS Nijmegen (NL); Wentink, Eva, 5591 MT Heeze (NL); Kho, Esther, 6709 VK Wageningen (NL)
(74) Representative: Körfer, Thomas

(57) **Abstract**

An optical sensor system (100) is provided. The optical sensor system comprises a track system (101), an optical sensor (102) operably coupled to the track system (101) comprising at least one emitter (103) and at least one detector (104), and a controller (105) operably coupled to the optical sensor (102) . In this regard, the optical sensor (102) is configured to perform measurements comprising at least one physiological parameter. Furthermore, the controller (105) is configured to actuate the optical sensor (102) to move the at least one emitter (103) and/or the at least one detector (104) along the track system (101) to at least one preferred location based on the at least one measured physiological parameter and/or at least one user parameter.

## Description

The invention relates to the measurement of physiological parameters, especially to perform passive acquisition of said physiological parameters.

The noninvasive techniques to measure physiological parameters are becoming popular due to their portability and easy to use nature. Embedding such noninvasive devices into stationary objects such as seats, toilet seats, beds allow for passive acquisition of the physiological parameter measurements during everyday living. Furthermore, such a device does not require the presence of a technical person during the measurement collection and processing.

For example, the document US 10,383,576 B2 discloses a synthetic aperture photoplethysmography (PPG) sensor system that comprises a group of matrix of contiguous PPG sensors located on a toilet seat. The group of matrix of contiguous PPG sensors are conditionally combined based on sensor measurements. Measurements associated with the conditional combinations are then used to determine a cardiac function of a toilet user.

However, due to the data acquisition from a large amount of sensors, the system is required to process a high volume of data, which results in high processing power consumption. Furthermore, the use of a large number of sensors also results in high energy/battery consumption due to their parallel/simultaneous operation. Moreover, the measurement location/position is limited due to the static positioning of the sensor matrix on the toilet seat.

Accordingly, an object of the invention is to provide an optical sensor system and a method that address the aforementioned limitations in order to facilitate reliable, accurate, and lightweight solution for passive acquisition of physiological parameters.

The object is solved by the features of the first independent claim for the optical sensor system and by the features of the second independent claim for the method. The dependent claims contain further developments.

According to a first aspect of the invention, an optical sensor system is provided. The optical sensor system comprises a track system or unit, an optical sensor operably coupled to the track system comprising at least one emitter, especially an optical emitter, and at least one detector, especially an optical detector, and a controller operably coupled to the optical sensor.

In this regard, the optical sensor, especially the at least one detector, is configured to perform measurements comprising at least one physiological parameter, such as but not limited to blood oxygen saturation, heart rate, respiration and cardiac output, and blood pressure. For instance, the at least one emitter may illuminate the tissue at a first location defined by its position on the track system and the at least one detector may measure the scattered and reflected light at a second location defined by its position on the track system, especially to optically measure the perfusion of blood within the tissue.

Furthermore, the controller is configured to actuate the optical sensor to move the at least one emitter and/or the at least one detector along the track system to at least one preferred location based on the at least one physiological parameter and/or at least one user parameter. The at least one user parameter may correspond to one or more physical attributes of a user, such as weight, biometric attributes including fingerprints, facial images, iris, and voice.

Therefore, the invention proposes a lightweight solution for passive acquisition of physiological parameters. Instead of using a plurality of sensors, the present invention facilitates a single sensor and a track or rail system along which the sensor can be moved. Due to the adaptability of the measurement locations/positions, an optimum measurement location/position can be identified using only one sensor, e.g. based on the quality of the detected signal.

The quality of the detected signal can be determined based on one or more signal traits, such as but not limited to signal-to-noise ratio (SNR), peak-to-peak interval, and amplitude, of the detected signal during the physiological parameter measurements. Additionally or alternatively, the quality of the detected signal can be determined based on the known signal quality indices for physiological parameters including but not limited to perfusion, kurtosis, and skewness.

The at least one preferred location can be an optimum measurement location on the track system. For instance, during a particular measurement scenario, the sensor may perform physiological parameter measurements to acquire physiological signals at different locations along the track system, e.g., iterative measurements at locations with defined increments of distance/length along the track system. Based on the quality of the acquired physiological signal, an optimum location on the track system can be identified, i.e., the location with the highest acceptable quality of the physiological signal. The sensor may then be moved or relocated at the optimum location for that measurement scenario, e.g., to resume the measurements and/or post-processing.

Preferably, the controller is configured to collect the measurements from the optical sensor, especially from the at least one detector. The controller is further configured to process the measurements to obtain processed results, and to actuate the optical sensor to move the at least one emitter and/or the at least one detector along the track system based on the measurements and/or the processed results.

The processing of the measurements may comprise preprocessing such as filtration, motion artefact removal and signal quality assessment, time-domain analysis, frequency-domain analysis, linear regression, physiological parameter estimation, and the like. The processed results may therefore render a qualitative verdict on the detected physiological parameters or signals. The verdict can be based on one or more signal traits and/or one or more signal quality indices as described before. In addition, the controller is configured to transmit the measurements and/or the processed results to a remote device, preferably a wireless remote device, e.g. smartphones, computers, personal digital assistants, or external signal processing/analysis devices.

Preferably, the optical sensor system comprises a user detection or recognition unit, e.g. comprising a weight sensor or load sensor, a biometric sensor, or a combination thereof. In this regard, the controller is configured to obtain a user detection or recognition signal comprising the at least one user parameter from the user detection unit and further to actuate the optical sensor based on the user detection signal, especially to move the at least one emitter and/or the at least one detector along the track system. Advantageously, user specific measurement locations can be detected or identified, which improves the measurement accuracy, reliability, and repeatability.

Preferably, the controller is configured to actuate the optical sensor, especially to move the at least one emitter and/or the at least one detector along the track system, to obtain the measurements at a plurality of locations along the track system in a defined sequence of steps. In addition, the controller is configured to generate a reference mapping based on the measurements and/or the processed results and/or the user detection signal.

For instance, the reference mapping may comprise or is a spatial distribution of a signal trait over a plurality of defined measurement locations, especially generated by mapping the measured signal or physiological parameter amplitudes and/or phases corresponding to the respective measurement locations along the track system.

Advantageously, user specific optimum measurement locations can be identified based on the generated reference mapping that indicates measured signal quality or intensity at different locations, which further improves the measurement accuracy, reliability, and repeatability.

Additionally or alternately, the controller is configured to actuate the optical sensor to obtain the measurements at a plurality of locations along the track system in a defined sequence of steps, and further to compare the measurements to a defined threshold to determine an optimum location on the track system based on the comparison and/or the user detection signal. For example, the threshold can be defined based on but not limited to a target SNR, amplitude, perfusion, kurtosis, skewness, and a ratio of difference in wavelengths of scatter light. Advantageously, optimum measurement locations for new users or a first time user of the sensor system can also be identified.

Preferably, the controller is configured to generate an alert signal based on the measurements and/or the processed results and/or the user detection signal, and further to transmit the alert signal to the remote device. In this regard, the alert signal comprises a tone and/or a text notification and/or a voice announcement. For instance, the controller may detect a linear translation in the positioning of the user based on the measurements and/or the processed results, and may notify the user via the alert signal whether the user and/or the sensor positioning is needed to be amended. The alert signal may comprise a tone and/or a text notification and/or a voice announcement. Advantageously, a feedback to the user regarding any anomalies during the measurement, e.g. due to false/incorrect positioning of the sensor system on target tissue, is also incorporated.

Preferably, the at least one emitter is configured to emit light with at least two different wavelengths and/or with at least two different intensities and/or with at least two different emission angles. Additionally or alternately, the at least one detector is configured to detect incident lights with at least two different wavelengths and/or with at least two different intensities and/or with at least two different incident angles. Advantageously, measurement reliability is further improved.

Preferably, the track system comprises a first track and a second track, whereby the at least one emitter is configured to move along the first track and the at least one detector is configured to move along the second track, especially when the optical sensor is being actuated by the controller.

In this regard, the first track and the second track are adjacent, preferably parallel to each other. Additionally or alternately, the at least one emitter and the at least one detector are configured to move simultaneously along the first track and the second track, respectively.

Alternately, the at least one emitter and the at least one detector are configured to move separately, especially at different start times and/or different speeds and/or different directions, along the first track and the second track.

Advantageously, the path of the light can be varied by varying the distance between the emitter and the detector to achieve the optimum measurement locations, e.g. based on the anatomical difference between users. For instance, if two users have differing amounts of subcutaneous fat, the respective positions of the emitter and the detector may be adjusted in order to adjust the penetration depth of the measured light.

Preferably, the track system is a linear track system or a semi-circular track system, especially an oval system, or a circular track system. In addition, the track system may comprise a mechanical actuator or an electro-mechanical actuator, e.g. a belt or ball screw, onto which the emitter and/or the detector are fixed and may be configured to be actuated by the controller so as to move the emitter and/or the detector. Alternately, the emitter and/or the detector may be provided with rotational means, e.g. motors, and are configured to be actuated by the controller in order to move the emitter and/or the detector along the track system.

Preferably, the optical sensor is a Photoplethysmography (PPG) sensor, preferably a reflective PPG sensor. Alternatively, the measurements comprising at least one physiological parameter are PPG measurements. In this regard, the at least one physiological parameter corresponds to one of the clinical PPG physiological parameters, such as but not limited to blood oxygen saturation, heart rate, respiration and cardiac output, blood pressure.

According to a second aspect of the invention, a toilet seat is provided. The toilet seat comprises at least one optical sensor system according to the first aspect of the invention, and a transparent cover configured to encompass the optical sensor system. In this regard, the optical sensor system is arranged on an upper surface of the toilet seat for contacting a user sitting on the toilet seat, especially for facilitating a physical contact and/or a near optical contact to the skin/tissue of the user. Advantageously, the present invention discloses a prospect of embedding the optical sensor system into static objects, such as the toilet seat, so as to facilitate passive acquisition of physiological parameters in a casual manner.

According to a third aspect of the invention, a method is provided. The method comprises a step of providing a track system, an optical sensor operably coupled to the track system comprising at least one emitter and at least one detector, and a controller operably coupled to the optical sensor. The method further comprises a step of performing measurements by the optical sensor comprising at least one physiological parameter. The method moreover comprises a step of actuating the optical sensor by the controller to move the at least one emitter and/or at least one detector along the track system to at least one preferred location based on the at least one measured physiological parameter and/or at least one user parameter.

It is to be noted that the method according to the third aspect corresponds to the optical sensor system according to the first aspect and its implementation forms. Accordingly, the method according to the third aspect achieves the same advantages and effects as the optical sensor system of the first aspect and its respective implementation forms.

Exemplary embodiments of the invention are now further explained with respect to the drawings by way of example only, and not for limitation. In the drawings:
- Fig. 1: shows a first exemplary embodiment of the optical sensor system according to the first aspect of the invention;
- Fig. 2: shows a second exemplary embodiment of the optical sensor system according to the first aspect of the invention;
- Fig. 3: shows a third exemplary embodiment of the optical sensor system according to the first aspect of the invention;
- Fig. 4: shows an exemplary embodiment of a device comprising the optical sensor system;
- Fig. 5A: shows a first exemplary embodiment of a toilet seat comprising the optical sensor system according to the second aspect of the invention;
- Fig. 5B: shows a second exemplary embodiment of a toilet seat comprising the optical sensor system according to the second aspect of the invention; and
- Fig. 6: shows an exemplary embodiment of the method according to the third aspect of the invention.

Reference will now be made in detail to the embodiments of the present invention, examples of which are illustrated in the accompanying drawings. However, the following embodiments of the present invention may be variously modified and the range of the present invention is not limited by the following embodiments. Reference signs for similar entities in different embodiments are partially omitted.

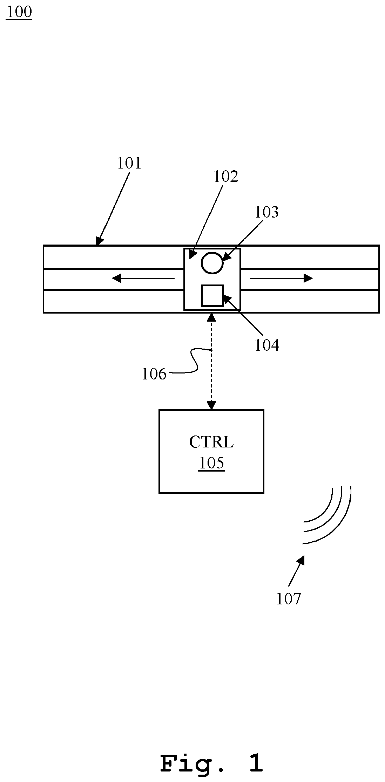

In Fig. 1, a first exemplary embodiment of the optical sensor system 100 according to the first aspect of the invention is illustrated. The optical sensor system comprises a track system or a rail system 101, and an optical sensor 102, especially a PPG sensor, comprising an emitter 103 and a detector 104, arranged on the track system 101.

The track system 101 and/or the optical sensor 102 has or have provision for indirect actuation, e.g. via belt or drive screw driven by motors, or direct actuation, e.g. via motors fixed at the optical sensor 102, in order to move the optical sensor 102 resp. the emitter 103 and the detector 104 along the track system 101. Although the track system 101 is illustrated as a linear track, it is to be understood that the track system 101 can also be implemented to comprise a semi-circular track or a circular track.

The optical sensor system 100 further comprises a controller (CTRL) 105 operably coupled to the track system 101 and/or to the optical sensor 102. The coupling between the controller 105 and the track system 101 and/or the optical sensor 102 is exemplarily illustrated by the dashed line 106. The controller 105 is configured to actuate the track system 101 and/or the optical sensor 102 in order to move the optical sensor 102, especially the emitter 103 and/or the detector 104, along the track system 101.

The optical sensor 102 preferably performs physiological parameter measurements on a contacted skin/tissue. More preferably, the optical sensor 102 optically measures the perfusion of blood within the tissue. In this regard, the emitter 103 may emit lights with different wavelengths, intensities, and/or incident angles in order to illuminate the tissue in a first location and the detector 104 may measure the scattered and reflected light in a second location. As such, the difference in the amount of reflected light over time can be used as an indication of changes in the absorption of light within the tissue.

The emitter 103 may comprise one or more light emitting diodes or laser diodes, and the detector 104 may comprise one or more photodiodes. The controller may comprise a processor, a microcontroller, a signal processing device, or a combination thereof. The controller may further comprise a memory to store the received measurements and/or processed results.

The controller 105 collects the measurements from the optical sensor 102, especially from the detector 104, processes the measurements thereby obtaining processed results. The controller may transmit the measurements and/or the processed results to a remote device, e.g. a smartphone, a computer, a cloud server and the like. The wireless transmission is exemplarily illustrated as signals 107 coming out of the controller 105.

The controller 105 may actuate the track system 101 and/or the optical sensor 102 to move the optical sensor 102 along the track system 101 in order to identify an optimum measurement location. Additionally or alternatively, the controller 105 may actuate the track system 101 and/or the optical sensor 102 to move the optical sensor 102 at a preferred measurement location on the track system 101.

For instance, based on the measurements collected from the optical sensor 102, especially based on the quality of the measured signal, the controller 105 may identify an optimum measurement location on the track system 101, i.e. the location with the highest acceptable quality of the measured signal, and may actuate the track system 101 and/or the optical sensor 102 to move the optical sensor 102 at the optimum measurement location.

Furthermore, the controller 105 may actuate the optical sensor 102 and/or the track system 101, especially to move the optical sensor 102 along the track system 101, to obtain the measurements at different and/or defined locations along the track system 101 in a defined sequence of steps. By way of this, the controller 105 is able to generate a reference map based on the measurements and/or the processed results.

For instance, a user upon first use of the optical sensor system 100 may undergo a scan in which the optical sensor 102, by means of the actuation from the controller 105, performs measurements at different and/or defined locations/positions along the track system 101. As such, the measurements can be translated as the reference map of area of the tissue scattering of the user.

When the user re-uses the optical sensor system 100, a fast scan can be performed, e.g. at a least number of locations/positions along the track to identify a match or at locations/positions where the signal quality is found acceptable according to the reference map.

Based upon the matching of the scan with the reference map, the controller 105 may determine if there is a linear translation in the positioning of the optical sensor system 100 or the user position, and may correct for this by actuating the optical sensor 102 to amend the position of the optical sensor 102 along the track system 101.

In this regard, the controller 105 may generate an alert signal based on the measurements and/or the processed results and/or the detection of the linear translation in the positioning, and may notify the user via a tone and/or a text notification and/or a voice announcement, for instance whether the user and/or the sensor positioning is needed to be amended.

The controller 105 may actuate the optical sensor 102 and/or the track system 101 to obtain the measurements at different and/or defined locations along the track system 101 in a defined sequence of steps. Afterwards, the controller 105 may compare the measurements to a defined threshold to determine an optimum location on the track system 101 based on the comparison, e.g. based upon a ratio of difference in wavelengths of scatter light.

In this way, the optical sensor 102 can be positioned at the optimum location, i.e. the best location and/or tissue with the highest acceptable signal quality, in a very fast manner.

Alternately, the generation of the reference map from the measurements and the technique of comparing the measurements to the defined threshold can be combined and be implemented altogether in order to enhance the measurement accuracy, reliability and repeatability.

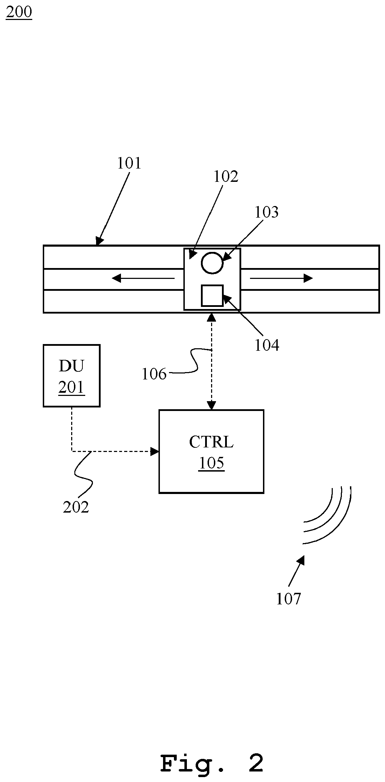

In Fig. 2, a second exemplary embodiment of the optical sensor system 200 according to the first aspect of the invention is illustrated. The optical sensor system 200 differs from the optical sensor system 100 in that the optical sensor system 200 additionally comprises a user detection unit (DU) or a user identification unit or a user identifier 201. The user detection unit 201 is operably coupled to the controller 105, where the coupling between the user detection unit 201 and the controller 105 is exemplarily illustrated by the dashed line 202.

The track system 101, the optical sensor 102, the emitter 103, and the detector 104 respectively correspond to the track system 101, the optical sensor 102, the emitter 103, and the detector 104 of the optical sensor system 100.

The user detection unit 201 may comprise a weight sensor, a biometric sensor, or a combination thereof. The user detection unit 201 may generate a user detection signal corresponding to a user of the optical sensor system 200. The controller 105 may obtain the user detection signal from the user detection unit 201 and may actuate the optical sensor 102 and/or the track system 101 based on the user detection signal, especially to move the optical sensor 102 resp. the emitter 103 and/or the detector 104 along the track system 101.

The controller 105 may use the user detection signal in conjunction with the measurements or processed results to identify the optimum location/position on the track system 101. The reference mapping scheme can also be augmented with the use of said user detection signal, since the controller 201 can make use of known optimum locations/positions based on the historical measurements of the user.

For instance, the controller 105 may obtain the user detection signal from the user detection unit and may cause the optical sensor 102 to move along the track system 101 in order to reposition the optical sensor 102 at a previously known optimum measurement location. This facilitates a fast, user-specific, and repeatable measurement at the optimum location/position.

Additionally or alternately, the controller 105 may cause the optical sensor 102 to move along the track system 101 to obtain the measurements at different and/or defined locations along the track system 101 in a defined sequence of steps, and may generate the reference map based on the user detection signal and the measurements and/or the processed results.

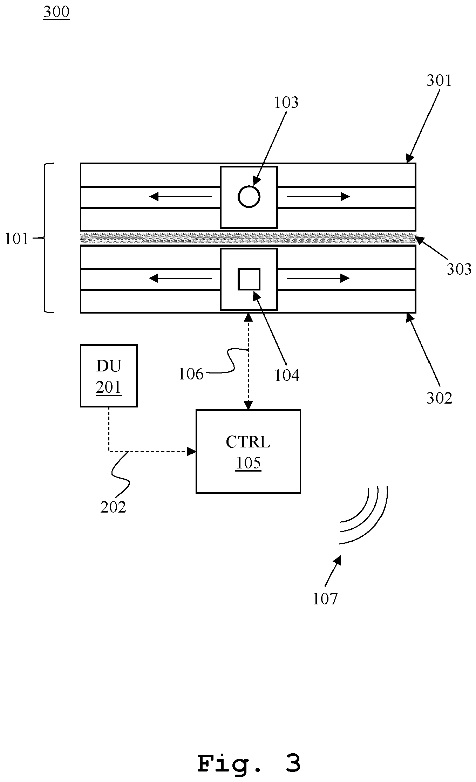

In Fig. 3, a third exemplary embodiment of the optical sensor system 300 according to the first aspect of the invention is illustrated. The optical sensor system 300 differs from the optical sensor system 200 in that the track system 101 of the optical sensor system 300 comprises a first track 301 and a second track 302. The controller 105 may be operably coupled to the first track 301 and the second track 302.

In this regard, the emitter 103 is arranged on the first track 301 and is configured to move along the first track 301, especially when the emitter 103 and/or the first track 301 is being actuated by the controller 105. Similarly, the detector 104 is arranged on the second track 302 and is configured to move along the second track 302, especially when the detector 104 and/or the second track 302 is being actuated by the controller 105.

The first track 301 and/or the emitter 103 has or have provision for indirect actuation, e.g. via belt or drive screw driven by motors, or direct actuation, e.g. via motors fixed at the emitter 103, in order to move the emitter 103 along the first track 301. The second track 302 and/or the detector 104 has or have provision for indirect actuation, e.g. via belt or drive screw driven by motors, or direct actuation, e.g. via motors fixed at the detector 104, in order to move the detector 104 along the second track 302.

Although the first track 301 and the second track 302 are illustrated as linear tracks, it is to be understood that the first track 301 and the second track 302 each can also be implemented as a semi-circular track or a circular track.

In this regard, the first track 301 and the second track 302 are adjacent tracks, preferably parallel to each other. The track system 101 may further comprise an optical barrier 303 in-between the first track 301 and the second track 302, especially along their lengths, especially to limit the scattered light from the emitter 103 towards the detector 104.

The emitter 103 and the detector 104 are arranged so as to move simultaneously along the first track 301 and the second track 302, respectively. Alternately, the emitter 103 and the detector 104 are arranged to move separately along the first track 301 and the second track 302, especially at different start times and/or different speeds and/or different directions.

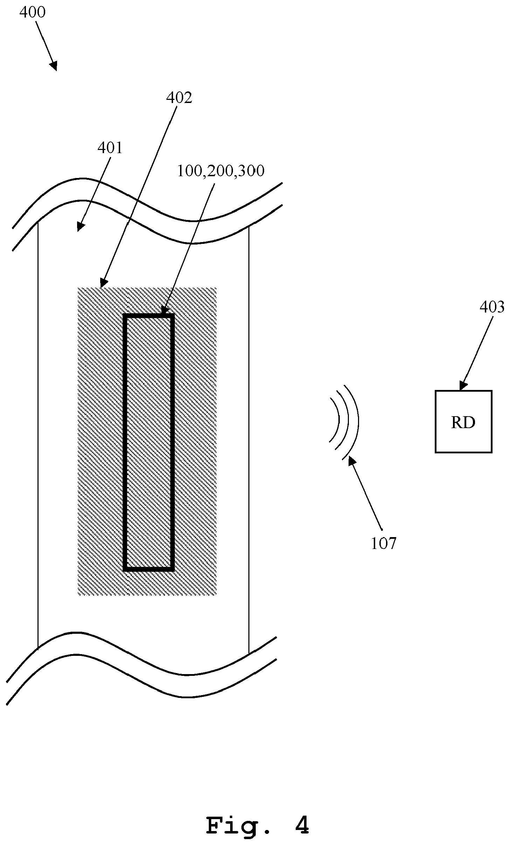

In Fig. 4, an exemplary embodiment of a device 400 comprising the optical sensor system 100, 200, 300 is illustrated. The device 400 comprises a support segment 401 with a sensor accommodation groove, the optical sensor system 100, 200, 300 embedded within the support segment 401, especially positioned in the sensor accommodation groove, and a transparent cover 402 encompassing the optical sensor system 100, 200, 300.

The support segment 401 can be a part of a seat, a bed, a wristwatch, an arm band, and so on. The sensor accommodation groove is formed such that the optical sensor system 100, 200, 300 positioned in the sensor accommodation groove can make near optical contact with the skin/tissue of a user of the device 400. The transparent cover 402 is preferably an optically transparent material that allows the transmission of light waves, especially with a wide spectral range.

The material of transparent cover 402 may include but is not limited to glass, films such as poly-methyl methacrylate (PMMA), polyethylene terephthalate (PET), and polyvinyl chloride (PVC).

The device 400, especially the optical sensor system 100, 200, 300 performs physiological measurements, e.g. PPG measurements, of a user of the device 400, and transmits the measurements to a remote device (RD) 403, especially wirelessly. The wireless transmission between the device 400 and the remote device 403 is exemplarily illustrated as signals 107 coming out of the device 400.

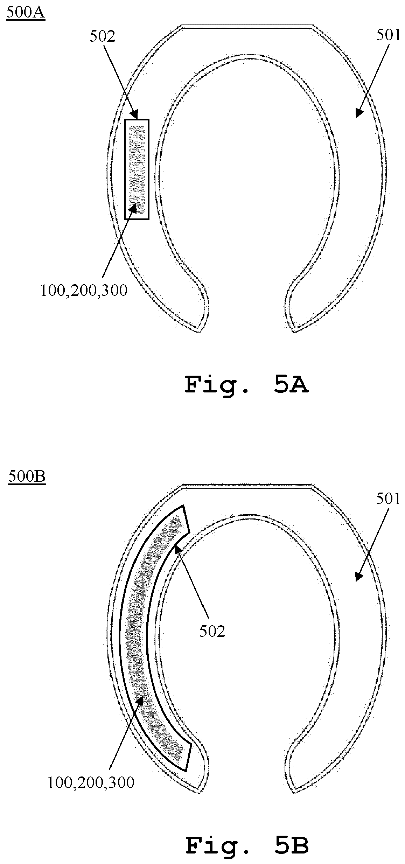

In Fig. 5A, a first exemplary embodiment 500A of a toilet seat 501 comprising the optical sensor system 100, 200, 300 according to the second aspect of the invention is illustrated. The toilet seat 501 comprises the optical sensor system 100, 200, 300, especially arranged on an upper surface of the toilet seat 501 for contacting a user sitting on the toilet seat 501. The toilet seat 501 further comprises a transparent cover 502 encompassing the optical sensor system 100, 200, 300.

The optical sensor system 100, 200, 300 can be arranged on the upper surface of the toilet seat 501 by means of a sensor accommodating groove. Alternately, the optical sensor system 100, 200, 300 can be partly implemented on the lower surface of the toilet seat 501, for instance by implementing the controller 105 on the lower surface and by implementing the track system 101 along with the optical sensor 102 on the upper surface, and by implementing the transparent cover 502 to encompass the track system 101.

The track system 101 is illustrated herein as a linear track system and the optical sensor 102, respectively the emitter 103 and/or the detector 104 move along the track system 101 in a linear fashion, especially along the upper surface of the toilet seat 501. In this regard, the user detection unit 201 may comprise additional weight sensors distributed along the toilet seat 501 in order to estimate the positioning of the user.

The transparent cover 502 is preferably an optically transparent material that allows the transmission of light waves, especially with a wide spectral range. The material of transparent cover 502 may include but is not limited to glass, films such as poly-methyl methacrylate (PMMA), polyethylene terephthalate (PET), and polyvinyl chloride (PVC) .

In Fig. 5B, a second exemplary embodiment 500B of a toilet seat 501 comprising the optical sensor system 100, 200, 300 according to the second aspect of the invention is illustrated. The second embodiment 500B differs from the first embodiment 500A in that the toilet seat 501 of the second embodiment 500B comprises a semi-circular track system 101.

Hence, the optical sensor 102, respectively the emitter 103 and/or the detector 104 move along the track system 101 in a semi-circular fashion, especially along the upper surface of the toilet seat 501. It is to be understood that the track system 101 can also be implemented in other shapes, e.g. an oval or a circular track system, depending on the shape and/or geometry of the toilet seat 501.

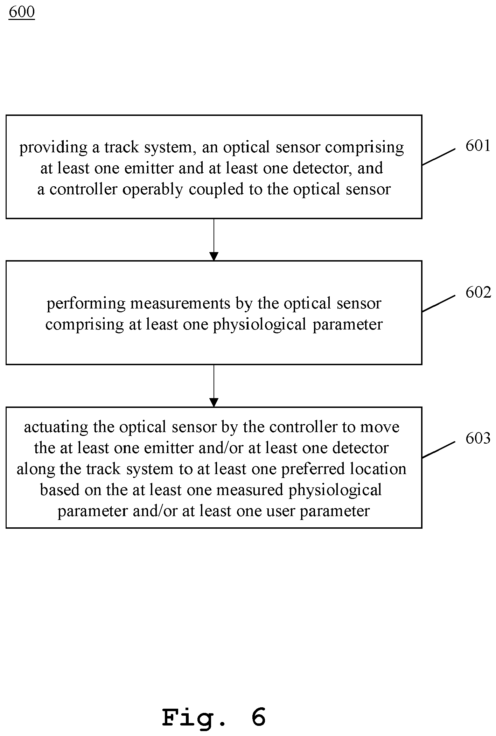

In Fig. 6, an exemplary embodiment of the method 600 according to the third aspect of the invention is illustrated. In a first step 601, a track system, an optical sensor operably coupled to the track system comprising at least one emitter and at least one detector, and a controller operably coupled to the optical sensor are provided. In a second step 602, measurements comprising at least one physiological parameter are performed by the optical sensor. In a third step 603, the optical sensor is actuated by the controller to move the at least one emitter and/or at least one detector along the track system, especially to move the at least one emitter and/or at least one detector to at least one preferred location on the track system based on the at least one measured physiological parameter and/or at least one user parameter.

Generally, in PPG measurements, intra-subject variations may occur due to different posture of a user on a stationary object embedded with the PPG sensor, such as a toilet seat, which may lead to poor repeatability between measurements. Furthermore, inter-subject variations may occur due to sub-optimal positioning of the sensor for different users with different anatomy. Moreover, the optimal path length for the emitter-detector distance may depend on the color of light, size/aperture of the detector, as well as on the location on the body where the measurements are performed.

The present invention solves the aforementioned limitations by introducing provisions to move the sensor over a defined measurement path, especially to move the emitter and the detector either simultaneously or separately. This especially allows PPG measurements at adaptable locations to identify an optimum measurement location, e.g. location of the sensor (or the respective locations of the emitter and the detector) on the track where the measurement signal quality is highly acceptable.

It is important to note that, in the description as well as in the claims, the word "coupled" implies that the elements may be directly connected together or may be coupled through one or more intervening elements. Furthermore, the word "comprising" does not exclude other elements or steps and the indefinite article "a" or "an" does not exclude a plurality. A single element or other unit may fulfill the functions of several entities or items recited in the claims. Moreover, the description with regard to any of the aspects is also relevant with regard to the other aspects of the invention.

Although the invention has been illustrated and described with respect to one or more implementations, equivalent alterations and modifications will occur to others skilled in the art upon the reading and understanding of this specification and the annexed drawings. In addition, while a particular feature of the invention may have been disclosed with respect to only one of several implementations, such feature may be combined with one or more other features of the other implementations as may be desired and advantageous for any given or particular application.

## Claims

1. An optical sensor system (100, 200, 300) comprising:
a track system (101),
an optical sensor (102) operably coupled to the track system (101) comprising at least one emitter (103) and at least one detector (104), and
a controller (105) operably coupled to the optical sensor (102),
wherein the optical sensor (102) is configured to perform measurements comprising at least one physiological parameter, and
wherein the controller (105) is configured to actuate the optical sensor (102) to move the at least one emitter (103) and/or the at least one detector (104) along the track system (101) to at least one preferred location based on the at least one measured physiological parameter and/or at least one user parameter.

2. The optical sensor system according to claim 1,
wherein the controller (105) is configured to collect the measurements from the optical sensor (102) and to process the measurements to obtain processed results, and further to actuate the optical sensor (102) to move the at least one emitter (103) and/or the at least one detector (104) along the track system (101) based on the measurements and/or the processed results, and
wherein the controller (105) is configured to transmit the measurements and/or the processed results to a remote device, preferably a wireless remote device (403).

3. The optical sensor system according claim 1 or 2,
wherein the optical sensor system further comprises a user detection unit (201), whereby the controller (105) is configured to obtain a user detection signal comprising the at least one user parameter from the user detection unit (201) and further to actuate the optical sensor (102) based on the user detection signal.

4. The optical sensor system according to any of claims 1 to 3,
wherein the controller (105) is configured to actuate the optical sensor (102) to obtain the measurements at a plurality of locations along the track system (101) in a defined sequence of steps, and further to generate a reference mapping based on the measurements and/or the processed results and/or the user detection signal.

5. The optical sensor system according to any of claims 1 to 4,
wherein the controller (105) is configured to actuate the optical sensor (102) to obtain the measurements at a plurality of locations along the track system (101) in a defined sequence of steps, and further to compare the measurements to a defined threshold to determine an optimum location on the track system (101) based on the comparison and/or the user detection signal.

6. The optical sensor system according to any of claims 1 to 5,
wherein the controller (105) is configured to generate an alert signal based on the measurements and/or the processed results and/or the user detection signal, and further to transmit the alert signal to the remote device (403).

7. The optical sensor system according to claim 6, wherein the alert signal comprises a tone and/or a text notification and/or a voice announcement.

8. The optical sensor system according to any of claims 1 to 7,
wherein the at least one emitter (103) is configured to emit light with at least two different wavelengths and/or with at least two different intensities and/or with at least two different emission angles.

9. The optical sensor system according to any of claims 1 to 8,
wherein the at least one detector (104) is configured to detect incident lights with at least two different wavelengths and/or with at least two different intensities and/or with at least two different incident angles.

10. The optical sensor system according to any of claims 1 to 9,
wherein the track system (101) comprises a first track (301) and a second track (302), whereby the at least one emitter (103) is configured to move along the first track (301) and the at least one detector (104) is configured to move along the second track (302).

11. The optical sensor system according to claim 10, wherein the first track (301) and the second track (302) are adjacent, preferably parallel to each other, and/or wherein the at least one emitter (103) and the at least one detector (104) are configured to move simultaneously along the first track (301) and the second track (302), respectively, or
wherein the at least one emitter (103) and the at least one detector (104) are configured to move separately, especially at different start times and/or different speeds and/or different directions, along the first track (301) and the second track (302).

12. The optical sensor system according to any of claims 1 to 11,
wherein the track system (101) is a linear track system or a semi-circular track system, especially an oval system, or a circular track system.

13. The optical sensor system according to any of claims 1 to 12,
wherein the optical sensor (102) is a Photoplethysmography sensor, preferably a reflective Photoplethysmography sensor.

14. A toilet seat (501) comprising at least one optical sensor system (100, 200, 300) according to claims 1 to 13, and a transparent cover (502) configured to encompass the optical sensor system (100, 200, 300),
wherein the optical sensor system (100, 200, 300) is arranged on an upper surface of the toilet seat (501) for contacting a user sitting on the toilet seat (501).

15. A method (600) comprising:
providing (601) a track system, an optical sensor operably coupled to the track system comprising at least one emitter and at least one detector, and a controller operably coupled to the optical sensor,
performing (602) measurements by the optical sensor comprising at least one physiological parameter, and
actuating (603) the optical sensor by the controller to move the at least one emitter and/or at least one detector along the track system to at least one preferred location based on the at least one measured physiological parameter and/or at least one user parameter.
